# FASCICULE DE BREVET EUROPEEN

(11) **EP 1 979 745 B1**
(45) Date de publication et mention de la délivrance du brevet: **15.10.2014**
(21) Numéro de dépôt: 07704031.9
(22) Date de dépôt: 19.01.2007
(51) Int. Cl.: G01N 33/564

(54) **MÉTHODE DE DIAGNOSTIC IN VITRO DE RÉPONSE IMMUNITAIRE AUTOIMMUNE PAR DÉTECTION D'ANTICORPS DIRIGÉS CONTRE L'ANTIGÈNE PENTRAXINE 3**
VERFAHREN ZUR IN-VITRO-DIAGNOSE EINER AUTOIMMUN-IMMUNANTWORT MITTELS NACHWEIS VON GEGEN DAS PENTRAXIN-3-ANTIGEN GERICHTETEN ANTIKÖRPERN
METHOD FOR THE IN VITRO DIAGNOSIS OF AN AUTOIMMUNE IMMUNE RESPONSE BY DETECTION OF ANTIBODIES DIRECTED AGAINST THE PENTRAXIN 3 ANTIGEN

(30) Priorité: 20.01.2006 FR 0600516
(43) Date de publication de la demande: 15.10.2008
(73) Titulaire: UNIVERSITE D'ANGERS, 49000 Angers (FR)
(72) Inventeur: JEANNIN, Pascale, F-49080 Bouchemaine (FR); DELNESTE, Yves, F-49080 Bouchemaine (FR); MANTOVANI, Alberto, I-20146 Milan (IT)
(74) Mandataire: Regimbeau
(86) Numéro de dépôt international: PCT/EP2007/050562
(87) Numéro de publication internationale: WO 2007/082945

(56) Documents cités:
- US-A1- 2005 043 230
- BOTTAZZI B ET AL: "Multimer Formation and Ligand Recognition by the Long Pentraxin PTX3" JOURNAL OF BIOLOGICAL CHEMISTRY, AMERICAN SOCIETY OF BIOLOCHEMICAL BIOLOGISTS, BIRMINGHAM,, US, vol. 272, no. 52, 26 décembre 1997 (1997-12-26), pages 32817-32823, XP002191385 ISSN: 0021-9258
- KRAVITZ MARTINE SZYPER ET AL: "Protective molecules - C-reactive protein (CRP), serum amyloid P (SAP), pentraxin3 (PTX3), mannose-binding lectin (MBL), and apolipoprotein A1 (Apo A1), and their autoantibodies: Prevalence and clinical significance in autoimmunity" JOURNAL OF CLINICAL IMMUNOLOGY, vol. 25, no. 6, novembre 2005 (2005-11), pages 582-591, XP002400269 ISSN: 0271-9142
- GARLANDA CECILIA ET AL: "Pentraxins at the crossroads between innate immunity, inflammation, matrix deposition, and female fertility" ANNUAL REVIEW OF IMMUNOLOGY, vol. 23, 2005, pages 337-366, XP002400270 ISSN: 0732-0582
- FAZZINI F ET AL: "PTX3 IN SMALL-VESSEL VASCULITIDES : AN INDEPENDENT INDICATOR OF DISEASE ACTIVITY PRODUCED AT SITES OF INFLAMMATION" ARTHRITIS AND RHEUMATISM, LIPPINCOTT, PHILADELPHIA, US, vol. 44, no. 12, décembre 2001 (2001-12), pages 2841-2850, XP009060523 ISSN: 0004-3591

## Description

L'invention concerne une méthode de diagnostic in vitro de réponse immunitaire autoimmune chez un sujet par détection dans un liquide biologique dudit sujet d'anticorps dirigés contre l'antigène pentraxine 3 (PTX3) caractérisée en ce que on détermine dans le sérum du sujet la présence d'anticorps dirigés contre l'antigène PTX3 (anticorps anti-PTX3), ainsi que l'utilisation de kits mettant en oeuvre la méthode.

### I. Etat de l'art

### 1. Les lymphocytes dans la réponse immune

### a. Les lymphocytes et la réponse immune physiologique anti-soi

Les cellules du système immunitaire, et en particulier les lymphocytes (B et T), ont pour rôle de détruire les agents étrangers au soi comme les microorganismes. Les lymphocytes expriment des récepteurs qui reconnaissent les agents microbiens. Ainsi par exemple, les lymphocytes T expriment le récepteur T et les lymphocytes B expriment les immunoglobulines, également appelées anticorps. Lorsqu'ils sont activés, les lymphocytes spécifiques des constituants des microorganismes libèrent des médiateurs qui ont pour fonction de détruire les microorganismes et/ou les cellules infectées par les microorganismes,

De façon schématique, ces médiateurs sont, pour les lymphocytes B, des immunoglobulines (ou anticorps). Ces anticorps se fixent aux microorganismes et/ou aux cellules infectées et facilitent leur élimination par d'autres cellules immunitaires telles que les macrophages, les polynucléaires, ou les cellules natural killer également appelées cellules NK. Les médiateurs facilitant l'élimination des microorganismes et produits par les lymphocytes T sont des cytokines et/ou des médiateurs toxiques.

Ainsi, la reconnaissance d'un microorganisme, (considéré comme du non-soi), par des lymphocytes spécifiques aboutit généralement à sa destruction par les cellules du système immunitaire.

### b. Processus auto-immuns et maladies auto-immunes

En plus des lymphocytes B et T qui ont des récepteurs qui reconnaissent des constituants du non-soi tels que les microorganismes, il existe chez tous les individus des lymphocytes qui reconnaissent également des constituants du soi. Dans des conditions physiologiques normales, ces lymphocytes ne sont pas activés. Ils sont maintenus, par différents mécanismes, dans un état de non réponse au soi, état appelé tolérance ou anergie.

Il peut arriver dans des conditions qui restent mal définies telles que par exemple une infection virale ou une prédisposition génétique, que ces lymphocytes soient activés. Il en résulte alors le développement d'une réponse immune anormale et délétère entraînant des destructions tissulaires. Le système immunitaire, et en particulier les lymphocytes, agissent comme si les cellules et/ou certaines molécules de l'individu étaient étrangères et vont donc mettre en place tout leur arsenal pour les détruire. En conséquence, les lymphocytes spécifiques du soi activés vont agir en libérant des anticorps et/ou des médiateurs toxiques.

Cette réaction des lymphocytes contre les constituants du soi caractérise les processus auto-immuns. Si cette réaction aboutit au développement d'une pathologie, on parle de maladie auto-immune. Néanmoins, un désordre de type auto-immun peut également être retrouvé dans de nombreuses autres pathologies telles que par exemple l'inflammation chronique, ou encore les pathologies associées à une lyse cellulaire importante.

### 2. Les auto anticorps

### a. Définitions

En cas de désordre immunologique de type auto-immun, les lymphocytes B spécifiques des constituants du soi sont activés et produisent des immunoglobulines spécifiques de ces constituants ; ces anticorps sont appelés auto anticorps. Les molécules et/ou structures du soi reconnues par des anticorps sont appelées auto antigènes. Les constituants du soi sont exprimés par les tissus, les cellules ou produites par les cellules de tous les individus. Certaines de ces molécules sont des protéines de structure, d'autres ont une fonction bien déterminée comme par exemple des molécules impliquées dans la coagulation et/ou dans la destruction/ élimination des constituants bactériens.

### b. Antigènes cibles des auto anticorps

Actuellement, une centaine de molécules, cibles d'auto anticorps ont été identifiées. Parmi celles-ci, citons à titre d'exemple :
- des anticorps anti-phospholipides qui sont dirigés contre deux protéines plasmatiques liées aux phospholipides anioniques : la β2-glycoprotéine I et la prothrombine,
- des anticorps anti-nucléaires solubles dirigés contre les antigènes Sm, Sc170, SSA, SSB, et Jol.,
- des anticorps anti-polynucléaires neutrophiles qui sont dirigés contre des protéines telles que la myélopéroxydase, la protéinase 3 et, de façon moins fréquente, contre la molécule BIP (bactericidal increasing protein), l'azurocidine, l'élastase, la cathepsine G.

### c. Rôle physiopathologique des auto anticorps

En plus d'un rôle essentiel dans le diagnostic d'un désordre immunitaire, certains auto anticorps peuvent jouer un rôle actif dans la pathogénicité de la maladie. A titre d'exemple, des dépôts d'immuns complexes (complexes auto anticorps / auto antigènes) circulants se déposent au niveau rénal et causent une glomérulonéphrite (inflammation aiguë du rein). Les anticorps anti-nucléosomes générés au cours du lupus érythémateux disséminé (LED) sont également directement impliqués dans le processus lésionnel rénal. Dans certains cas particuliers, la présence d'auto anticorps altère la fonction de l'auto antigène et a des conséquences physiopathologiques. Néanmoins, à l'heure actuelle, bien que l'on soupçonne une corrélation entre la présence et le taux d'auto anticorps et l'atteinte des organes, pour la majorité des auto anticorps de spécificité connue, leur rôle physiopathologique n'est pas connu.

### d. Diagnostic immunologique permettant de rechercher les auto anticorps

Actuellement les techniques les plus utilisées en routine dans les laboratoires de biologie ou d'Immunologie pour mettre en évidence un processus auto-immun consistent à rechercher dans les sérums des patients la présence d'auto anticorps.

La présence d'auto anticorps capables de se fixer à différents tissus, humains ou non, à des cellules ou des protéines est analysée. Des tissus non humains sont parfois utilisés quand il a été préalablement démontré que la cible des autoanticorps est conservée à travers les espèces.

Les techniques utilisées pour rechercher des auto anticorps lorsque l'on utilise comme source d'auto antigènes des tissus ou des cellules sont respectivement des techniques d'immuno-histochimie et d'immuno-cytochimie : des coupes de tissus ou des cellules sont mises en présence du sérum testé à différentes dilutions. Après incubation puis lavage, la recherche d'immunoglobulines qui reconnaissent les tissus ou cellules est mise en évidence en utilisant un anticorps anti-immunoglobuline humaine couplé à une molécule qui permet sa détection tel que par exemple un fluorochrome, ou un complexe enzyme-substrat.

Lorsque les molécules reconnues par des auto anticorps sont connues, d'autres techniques sont utilisées : ELISA, Immuno-Dot et/ou immunoempreintes. Dans les deux premiers cas, la molécule (auto-antigène) est adsorbée, respectivement, sur une plaque en polystyrène ou sur une membrane. La plaque ELISA ou la membrane sont incubées avec le sérum et les anticorps présents dans le sérum qui se sont fixés sur la cible sont révélés avec un anticorps anti-imununoglobuline humaine couplé à une molécule qui permet sa détection tel que par exemple un fluorochrome, ou un complexe enzyme-substrat. Dans le cas de l'immuno-empreinte, un extrait protéique total issu d'une cellule ou une molécule purifiée migrent dans un gel de polyacrylamide. Les molécules ainsi séparées selon leur poids moléculaire sont ensuite transférées sur une membrane qui subit le même protocole que celui décrit ci-dessus.

### e. Intérêt diagnostique et pronostique de la détection d'auto anticorps

L'intérêt de la mise en évidence d'auto anticorps réside dans l'indication de l'existence d'une dysimmunité, c'est-à-dire d'une réponse immune anormale qu'elle permet. L'identification d'auto anticorps n'a d'intérêt diagnostic que couplé à des informations cliniques. En effet, la génération d'auto anticorps peut précéder les manifestations cliniques. La présence d'auto anticorps contre certains auto antigènes est majoritairement associée à certaines pathologies dites immunitaires telles que par exemple les auto anticorps anti-nucléaire et le LED. Les résultats biologiques sont généralement essentiels en complément du tableau clinique pour faciliter la conduite à tenir en thérapeutique, surtout lorsque ce tableau est atypique, ce qui est souvent le cas lorsque la maladie est détectée précocement. En effet, les traitements actuels tels que les immunosuppresseurs et les corticoïdes sont efficaces mais agressifs. La présence d'auto anticorps, leur spécificité et le taux de ces auto anticorps est donc en général essentiel au clinicien pour évaluer le ratio bénéfice/risque des traitements.

### f. Intérêt actuel de la recherche de nouvelles cibles des auto anticorps

Actuellement, la recherche de nouvelles cibles des auto anticorps apparaît comme une niche de choix dans le domaine des biotechnologies. Les maladies auto immunes représentent la troisième cause de morbidité dans les pays industrialisés. Le vieillissement de la population contribue à cette augmentation. La recherche de nouvelles cibles permet un diagnostic rapide et précoce d'une réponse immunitaire autoimmune ainsi que de compléter les panels de cibles d'auto antigène utilisés dans les laboratoires. Il existe actuellement une réelle volonté de disposer de tests diagnostiques complémentaires à ceux existants afin de bénéficier d'informations plus précoces et mieux adaptées pour suivre l'évolution de la maladie. En effet, un traitement précoce permet d'éviter les complications et de limiter le coût thérapeutique de ces pathologies chroniques. Notons que des techniques de diagnostique rapide apparaissent également comme essentielles lorsqu'il y a engagement du pronostique vital tel que par exemple lors d'atteinte rénale ou pulmonaire.

Ceci est justement l'objet de la présente invention.

### II. L'invention

En effet, les inventeurs ont pu trouver de quoi répondre à cette attente en montrant pour la première fois la présence d'auto-anticorps, dirigés contre la molécule pentraxine 3 (PTX3), également appelée TNF-inducible gene 14 (TSG-14), dans des liquides biologiques de sujets atteints de réponse immunitaire autoimmune.

De ce fait, la présente invention concerne une méthode de diagnostic in vitro de réponse immunitaire autoimmune chez un sujet par détection dans un liquide biologique dudit sujet d'anticorps dirigés contre l'antigène pentraxine 3 (PTX3).

### I. La pentraxine 3, un médiateur de l'immunité innée

### a. Les récepteurs de l'immunité innée

Les cellules de l'immunité innée sont impliquées dans la reconnaissance rapide des microbes ; leur activation permet de contrôler la propagation des microbes, via notamment la production de médiateurs microbicides ainsi que le développement d'une réponse immune spécifique. Afin d'échapper à la reconnaissance par les cellules immunitaires innées qu'ils rencontrent, les microbes génèrent de nombreuses mutations afin d'augmenter l'hétérogénéité de leurs composants. Afin de contrecarrer cette stratégie d'échappement de reconnaissance, les cellules immunitaires innées ont sélectionné des récepteurs capables de reconnaître des structures fortement conservées chez les microorganismes et nécessaires à la physiologie des micro-organismes. Ces structures conservées tels que les lipopolysaccharides, les ARN double brins et les séquences CpG sont regroupées sous le terme de Pathogen-Associated Molecular Pattem ou PAMPs. Par définition, les PAMPs sont distincts du soi, partagés par les grands groupes de pathogènes et sont essentiels à leur survie. Ils constituent de véritables signatures moléculaires des microbes et leur reconnaissance est à l'initiation de la réponse immune anti-microbienne.

Les récepteurs impliqués dans la reconnaissance des PAMPs sont appelés récepteurs de l'immunité innée ou Pattern-Recognition Receptors (PRRs). Les PRRs reconnaissent un large spectre de constituants microbiens comme des sucres, des protéines, des lipides et des acides nucléiques (Medzhitov et Janeway, 2000 ; Janeway et Medzhitov, 2002). Les PRRs sont exprimés par les cellules immunitaires innées soit au niveau intracellulaire, membranaire ou dans le milieu extracellulaire.

Les PRRs sont distingués selon leur fonction biologique:
- les PRRs de reconnaissance, impliqués dans la détection et/ou l'internalisation des micro-organismes par les cellules immunitaires innées et,
- les PRRs de signalisation, impliqués dans l'activation des cellules immunitaires par les micro-organismes.

Les PRRs de reconnaissance membranaires appartiennent, entre autre, à la famille des récepteurs d'épuration, des récepteurs au mannose, des lectines de type C et de la famille des intégrines. Les PRRs de reconnaissance solubles, encore appelés opsonines, appartiennent à la famille des collectines (van de Wetering *et al,* 2004), à la famille des ficolines (Matsushita & Fujita, 2002) et à la famille des pentraxines (Garlanda *et al,* 2005). Les PRRs de reconnaissance solubles ont pour rôle de reconnaître, de se fixer et de favoriser l'élimination des microbes par les cellules phagocytaires.

La superfamille des pentraxines (PTX) regroupe des molécules très conservées durant l'évolution caractérisées par la présence d'un domaine «pentraxine» dans la région C-terminale. Elle comprend plusieurs membres dont :
- la protéine C-réactive (C-reactive protein ou CRP, encore appelée PTX1) et le sérum amyloide P (encore appelée PTX2). CRP et SAP sont des protéines de la phase aiguë produites par le foie en réponse à des stimuli pro-inflammatoires comme IL-6. CRP et SAP se lient à une grande variété de molécules incluant le soi (composante Clq du complément) (Agrawal & Volanakis, 1994 ; le soi modifié (les cellules apoptotiques) (Gershov *et al,* 2000 ; Bijl *et al,* 2003) et le non-soi (bactéries et virus) (Hind *et al, 19*84); cependant, les fonctions de CRP et SAP restent mal connues,
- la molécule PTX3, encore appelée TSG-14 (TNF-stimulated gene 14) a été identifiée comme une molécule dont la synthèse est fortement augmentée dans les fibroblastes et les cellules endothéliales en réponse à une stimulation par le TNFα ou l'IL- 1β (Breviario *et al,* 1992 ; Lee *et al*, 1993). PTX3 est le prototype des pentraxines longues : le domaine C-terminal de PTX3 est homologue (17% d'identité) à CRP entier et contient un domaine N-terminal complémentaire sans homologie avec aucune autre molécule (Garlanda *et al*, 2005).

### b. La molécule PTX3

La molécule PTX3 est produite par de nombreux types cellulaires tels que les cellules endothéliales, les fibroblastes, les chondrocytes, les myocytes, les phagocytes mononucléés, les cellules dendritiques et les cellules épithéliales en réponse à un stimulus pro-inflammatoire ou à un composant microbien (Breviario *et al*, 1992 ; Abderrahim-Ferkoune *et al*, 2003 ; Alles *et al,* 1994 ; Doni *et al,* 2003 ; Goodman *et al,* 2000 ; Vouret-Craviari *et al,* 1997 ; Nauta *et al,* 2005 ; Garlanda *et al*, 2005). La molécule PTX3 est produite sous forme d'un multimère constitué de 10 à 20 sous-unités (Bottazzi *et al,* 1997).

Au contraire de CRP et SAP, de nombreuses fonctions ont été décrites pour la molécule PTX3:
- PTX3 se lie à la composante Clq du complément ; cette fixation peut soit activer ou inhiber la voie classique du complément en fonction de la structure soluble ou immobilisée de Clq (Nauta *et al,* 2003),
- PTX3 se lie aux cellules apoptotiques et favorise leur élimination par les cellules phagocytaires (Rovere *et al,* 2000),
- PTX3 se fixe à certains pathogènes comme *Salmonella typhimurium, Pseudomonas aeruginosa* et *Aspergillus fumigatus,* facilitant leur ingestion par les cellules phagocytaires (Garlanda *et al,* 2002 ; Diniz *et al,* 2004).

Les souris transgéniques surexprimant PTX3 ont une survie augmentée dans un modèle d'endotoxémie (Dias *et al,* 2001). Au contraire, les souris déficientes en PTX3 présentent une sensibilité accrue à une aspergillose pulmonaire (Garlanda *et al,* 2002). L'ensemble de ces données montre que la molécule PTX3 se comporte comme un PRR soluble jouant un rôle essentiel dans la reconnaissance du soi modifié et du non soi.

### II. Description de l'invention

Un premier aspect de la présente invention concerne une méthode de diagnostic in vitro de réponse immunitaire autoimmune chez un sujet par détection dans un liquide biologique dudit sujet d'autoanticorps dirigés contre l'antigène pentraxine 3 (PTX3) caractérisée en ce que on détermine dans les liquides biologiques du sujet la présence d'anticorps dirigés contre l'antigène PTX3 (anticorps anti-PTX3) et qu'on en conclue l'existence d'une réponse immunitaire autoimmune chez le sujet.

Par réponse immunitaire autoimmune, on entend désigner l'existence de lymphocytes B producteurs d'anticorps dirigés contre des molécules du soi.

Par « anticorps anti-PTX3 », on entend désigner selon la présente invention toute molécule comprenant un « paratope » capable de se lier spécifiquement à la protéine PTX3. Par « anticorps anti-PTX3 », on entend également désigner selon la présente invention une population homogène de molécules comprenant toutes le même « paratope » capable de se lier spécifiquement à la protéine PTX3.

On entend par « paratope » le site de combinaison antigénique compris dans le fragment Fab d'un anticorps qui est localisé dans les régions hypervariables ou CDR des domaines variable V_{H} et V_{L} de la chaîne lourde et de la chaîne légère d'une immunoglobuline.

Selon la présente invention, on détermine dans un liquide biologique du sujet le taux d'anticorps dirigés contre l'antigène PTX3 et en le comparant avec le taux d'anticorps dirigés contre l'antigène PTX3 de référence défini à partir de sérums de sujets sains, on détermine l'existence d'une réponse immunitaire autoimmune chez le sujet.

De manière préférée selon la présente invention, la présence et/ou le taux d'anticorps dirigés contre PTX3 est déterminé par la mise en évidence d'une liaison entre l'antigène PTX3 et l'anticorps anti-PTX3.

La réaction de liaison antigène - anticorps est due à l'interaction entre les épitopes de l'antigène et les paratopes de l'anticorps. Elle fait intervenir quatre types de liaisons non covalentes (des liaisons hydrogènes, des liaisons électrostatiques, des liaisons hydrophobes et les forces de Van der Waals).

Selon la présente invention, la mise en évidence d'une liaison entre l'antigène PTX3 et l'anticorps anti-PTX3 est réalisée préférentiellement par immobilisation de l'antigène PTX3 sur un support solide, par réactions de précipitation en milieu liquide et/ou immunoprécipitation, et/ou par réaction de précipitation en gel.

Par réaction de précipitation en milieu liquide, on entend désigner selon la présente invention une réaction qui consiste à répartir des quantités égales d'une solution d'antigène PTX3 avec des dilutions croissantes d'un liquide biologique, de préférence un sérum immun.

La zone d'équivalence (qui est le point où la courbe atteint son maximum) correspond à la formation d'une liaison antigène-anticorps.

La fixation d'un antigène PTX3 selon l'invention sur un support solide peut être réalisée par des techniques bien connues de l'homme du métier. Le support peut se présenter sous des formes diverses, y compris sous forme de bandes ou de particules telles que des billes. La surface du support peut être polyfonctionnelle ou susceptible d'être polyfonctionnalisée de manière à fixer l'antigène PTX3 via des interactions covalente ou non covalente qui peuvent être spécifiques ou non spécifiques.

A titre illustratif, le support sur lequel est immobilisé l'antigène PTX3 peut être un matériau insoluble dans l'eau poreux ou non poreux. Le support peut être hydrophile ou susceptible d'être rendu hydrophile et comprend les poudres inorganiques telles que la silice, le sulfate de magnésium et l'aluminium ; les matériaux polymères naturels, en particulier les matériaux cellulosiques et les matériaux dérivés de la cellulose ; des polymères naturels ou synthétiques tels que la nitro cellulose, l'acétate de cellulose, le poly (chlorure de vinyle), le polyacrylamide, le dextran réticulé, l'agarose, le polyacrylate, le polyéthylène, le polypropylène, le poly(4-méthylbutène), le polystyrène, le polyméthacrylate, le poly (téréphtalate d'éthylène), le Nylon, le poly (butyrate de vinyle), certains types de verre tels que le Bioglass, ou des céramiques.

Le support solide pour l'immobilisation de l'antigène PTX3 est préférentiellement de type plastique ou polypropylène pour un dosage ELISA ou RIA, de type membrane pour un dosage Western blot ou Dot blot, de type billes ou de type mousse.

Par ELISA (*Enzyme Linked ImmunoSorbent Assay*), on entend désigner selon la présente invention est un test immunologique destiné à détecter et/ou doser les anticorps anti-PTX3 dans un liquide biologique, par marquage immunoenzymatique.

Par RIA (Radioimmunology assay) on entend désigner selon la présente invention un test radio immunologique destiné à détecter et/ou doser les anticorps anti-PTX3 dans un liquide biologique sur le même principe que l'ELISA, mais par marquage radioimmunologique.

Par dosage fluorimétrique, on entend désigner selon la présente invention la numération de particules, par exemple des billes, présentant à leur surface l'antigène PTX3, et auquel se seront liés des anticorps anti-PTX3 éventuellement présents dans un échantillon à tester. Les anticoprs anti-PTX3 fixés seront détectés à l'aide d'anticorps anti-immunoglobulines humaines couplées à un fluorochrome. La mesure de la quantité d'anticorps anti-PTX3 est fonction de l'intensité de fluorescence. Ceci est réalisé à l'aide d'un analyseur de fluorescence ou d'un analyseur de cellules : cytomètre de flux ou FACS (fluorescence activated cell sorter).

Par Western blot, ou immunotransfert, on entend désigner selon la présente invention une technique où l'antigène PTX3 est séparé par électrophorèse sur gel de polyacrylamide, puis transféré électrophorétiquement sur une membrane (de nitrocellulose, par exemple). Le dépôt sur la membrane de l'échantillon de liquide biologique à tester permet alors de lier des anticorps anti-PTX3, qui seraient présents dans l'échantillon, à l'antigène PTX3, suivi d'une détection par un second Anticorps marqué par un isotope, un fluorochrome ou une enzyme.

Par Dot blot on entend désigner selon la présente invention une technique d'absorption qui consiste à déposer des protéines sur une membrane de nitrocellulose sous forme de points puis à effectuer les étapes d'un immunobuvardage classique.

De plus, la présence et/ou le taux des anticorps anti-PTX3 est déterminée selon la présente invention en utilisant un substrat chromogène, par chimioluminescence, par fluorescence, ou par radiomarquage.

Par substrat chromogène, on entend désigner le substrat chromogène d'une enzyme qui permet d'obtenir en final, une réaction colorée qui est détectée à l'aide d'un spectrophotomètre. A titre d'exemple, on peut citer X-gal/OPTG et salmon-gluc/O-Me-b-Gluc, OPD, ABTS.

Par chimioluminescence on entend désigner une réaction chimique accompagnée d'une émission lumineuse, la mesure de la lumière émise permettant de quantifier l'un des réactifs, si la quantité de l'autre réactif est connue. A titre d'exemple on peut citer l'oxydo-réduction du luminol (3-aminophthalhydrazide) avec l'eau oxygénée, par exemple, ou un quelconque hydroxyde.

Par fluorescence on entend désigner l'utilisation d'une molécule fluorescente telle que les molécules décrites par ICHINOSE et al. (1991) ou encore des dérivés d'isothiocyanate fluorescents, la phycoerithrine,l'isothiocyanate de rhodamine, le chlorure de dansyle ou encore le composé XRITC, la protéine GFP (Green Fluorescent Protein ) du poisson Aequorea Victoria et ses nombreux dérivés, ou encore la protéine YFP( Yellow Fluorescent Protein ) ainsi que la protéine luciférase.

Par radiomarquage on entend désigner un marquage par substance radioactive. La substance radioactive peut être marquée, par exemple par un isotope choisi parmi[³H], [³²P] et[¹²⁵I].

Selon la présente invention, le dosage ELISA ou RIA permet de quantifier le taux d'anticorps anti-PTX3 et le dosage de type Dot blot ou Western blot permet de déceler des taux d'anticorps anti-PTX3 plus élevés par rapport au taux d'anticorps anti-PTX3 de référence.

Par « taux d'anticorps anti-PTX3 de référence » on entend désigner selon la présente invention le taux d'anticorps anti-PTX3 obtenu à partir du sérum d'un sujet sain, d'un ensemble de sérum de sujets sains ou défini de façon arbitraire.

Par sujet sain, on entend désigner selon la présente invention un sujet ne présentant pas de réponse immunitaire autoimmune.

Par « taux d'anticorps anti-PTX3 défini de façon arbitraire », on entend désigner selon la présente invention tous moyens qui permettront de reproduire la valeur moyenne obtenue avec un pool de sujets sains.

De manière encore préférée, la réaction de précipitation en gel est choisie parmi les réactions de type immunodiffusion radiale, immunodiffusion double d'Ouchterlony, immunoélectrophorèse et électrophorèse en fusée.

Par Immunodiffusion radiale, également appelée technique de Mancini, on entend désigner selon la présente invention une réaction de précipitation en gel qui consiste à incorporer une solution d'antigène PTX3 dans la gélose et à déposer un liquide biologique susceptible de contenir des anticorps anti-PTX3 dans des puits. A l'équilibre il se forme un anneau de précipitation dont le carré du diamètre est proportionnel à la concentration des anticorps anti-PTX3. La concentration est exprimée par référence à une courbe standard avec un anticorps anti-PTX3 de concentration connue.

Par immunodiffusion double d'Ouchterlony on entend désigner selon la présente invention une réaction de précipitation en gel réalisée de la façon suivante :
les solutions d'antigène PTX3 et d'anticorps anti-PTX3 sont déposées dans des puits percés à distance les uns des autres dans un gel d'agarose. Les molécules diffusent dans le gel en fonction de leur taille et forment des lignes de précipitation pour chaque système d'antigène et d'anticorps.

Chaque ligne de précipitation correspond à la zone d'équivalence respective, c'est-à-dire à la formation d'une liaison antigène-anticorps.

Cette méthode permet l'analyse d'un liquide biologique et l'identification d'anticorps anti-PTX3.

Par immunoéléctrophorèse, on entend désigner selon la présente invention une réaction de précipitation en gel qui met en jeu une séparation des protéines par électrophorèse dans un gel d'agarose suivie d'une double diffusion contre des anticorps spécifiques selon une direction perpendiculaire à l'axe de migration électrophorétique. Chaque zone d'équivalence correspond à un précipité antigène-anticorps qui se traduit par un arc de précipitation. L'immunoélectrophorèse permet de caractériser ou d'identifier des anticorps anti-PTX3 mais ce n'est pas une méthode quantitative.

Par électrophorèse en fusée on entend désigner selon la présente invention une réaction de précipitation en gel dans laquelle l'antigène PTX3 incorporé dans le gel d'agarose est immobile (grâce au pH du gel), et l'anticorps anti-PTX3 chargé négativement migre sous l'action d'un champ électrique.

L'arc de précipitation résultant à la forme d'une fusée dont la hauteur est proportionnelle à la concentration de l'anticorps anti-PTX3.

Par liquide biologique on entend désigner selon la présente invention le sang, le sérum, le plasma, la lymphe, l'urine, la salive, le liquide céphalorachidien préférentiellement le sérum.

Dans un mode de réalisation préféré de l'invention, la méthode de diagnostic selon la présente invention est un dosage ELISA et elle comprend les étapes suivantes :
a) incuber le sérum du sujet avec les antigènes PTX3 fixés sur un support solide
b) laver les anticorps du sérum non fixés aux antigènes PTX3 du support solide
c) ajouter des anticorps anti-immunoglobuline couplés à un marqueur, lesdits anticorps anti-immunoglobuline étant capables de reconnaître les anticorps du sérum
d) laver les anticorps anti-immunoglobuline non fixés au support solide
e) détecter et/ou quantifier le marqueur lié au support solide et corréler avec la présence et/ou la quantité d'anticorps du sérum.

La concentration est variable, et est à déterminer suivant le support et la source de PTX3.

Les antigènes PTX3 selon la présente invention sont choisis parmi la PTX3 entière d'origine humaine, animale, ou synthétique, un ou plusieurs fragments de PTX3 d'origine humaine, animale ou synthétique, et une molécule homologue à PTX3, préférentiellement de la famille des pentraxines, et/ou présentant des homologies de séquences primaires, secondaires ou tertiaires importantes.

Les antigènes PTX3 selon la présente invention sont obtenus à partir d'un système recombinant procaryote par exemple dans une souche d'Escherichia coli, d'un système recombinant eucaryote, de préférence des cellules CHO et la lignée N50, de la purification à partir des cellules, tissus, ou liquides biologiques humains ou animaux, ou par synthèse chimique, de préférence synthèse peptidique.

Par synthèse peptidique, on entend désigner selon la présente invention la synthèse en phase solide de peptides de PTX3.

Les anticorps anti-immunoglobuline selon la présente invention sont sélectionnés dans le groupe comprenant des anti immunoglobuline G, des anti immunoglobuline A, des anti immunoglobuline M, des anti immunoglobuline D, et des anti immunoglobuline E.

Dans ce mode de réalisation préféré de l'invention, le support solide consiste en des microbilles ou en des plaques de microtitre, tel que les plaques ELISA. et le marqueur est sélectionné dans le groupe comprenant les marqueurs fluorescents, chimioluminescents, enzymatiques et radioactifs.

Plus particulièrement, dans ce mode de réalisation préféré de l'invention le support solide est une plaque ELISA et comprend les étapes suivantes :
a) incuber le sérum du sujet avec les antigènes PTX3 fixés sur la plaque ELISA
b) laver les anticorps du sérum non fixés aux antigènes PTX3 de la plaque ELISA
c) ajouter des anticorps anti-immunoglobuline couplés à une enzyme, lesdits anticorps anti-immunoglobuline étant capable de reconnaître les anticorps du sérum
d) laver les anticorps anti-immunoglobuline non fixés à la plaque ELISA
e) ajouter le substrat soluble correspondant à l'enzyme
f) lire les valeurs d'absorbances des puits de la plaque ELISA dans un lecteur ELISA, à une longueur d'onde appropriée et corréler avec la présence et/ou la quantité d'anticorps du sérum.

De plus, les enzymes et les substrats solubles correspondant selon ce mode de réalisation préféré de l'invention sont sélectionnés dans le groupe comprenant :
- La phosphatase alcaline et le substrat soluble 4-NitroPhényl Phosphate (PNPP)
- La peroxydase et le substrat soluble orthophenylène diamine (OPD)
- La β-galactosidase et le substrat soluble 2-nitrophenyl β-galactoside (ONPG)
- La glucose 6-phosphate dehydrogenase et le substrat soluble glucose-6-phosphate (G6P).
- La biotine et le substrat soluble streptavidine couplé à la peroxydase et le substrat ABTS ou OPD de la peroxydase.

Selon la présente invention, le sujet est préférentiellement un mammifère, encore plus préférentiellement un être humain.

Selon la présente invention, les anticorps anti-immunoglobuline sont préférentiellement des anticorps anti-immunoglobuline humaine.

Selon la présente invention, le diagnostic d'une réponse immunitaire autoimmune peut être associé à l'existence ou à la prédiction d'une maladie auto-immune chez le sujet. De manière préférée, les maladies auto-immunes sont sélectionnées dans le groupe comprenant le syndrome de Gougerot-Sjögren, le diabète de type 1, la gammapathie monoclonale, la granulomatose de Wegener, le lupus érythémateux disséminé, la maladie athéromateuse, la maladie de Crohn, la maladie de Horton, la maladie de Reiter (syndrome conjonctivo-urétro-synovial), la polyarthrite rhumatoïde, la rectocolite hémorragique, le rhumatisme psoriasique, la sarcoïdose, la sclérodermie, la sclérose en plaques, et les dermatites bulleuses auto-immunes, la maladie de Basedow (hyperthyroïdie), la thyroïdite chronique de Hashimoto (hypothyroïdie), le symdrôme de Goodpasture, le pemphigus, la myasthénie, le diabète par insulinorésistance, l'anémie hémolytique auto-immune, la purpura thrombocytopénique auto-immun, polymyosite et dermatomyosite, l'anémie de Biermer, la glomérulonéphrite, certaines stérilités, la périartérite noueuse et le syndrome de Churg et Strauss, la maladie de Still, la polychrondite atrophiante, la maladie de Behçet, la spondylarthrite.

La méthode de diagnostic d'une réponse immunitaire autoimmune associé à l'existence d'une maladie auto-immune chez un sujet selon un aspect de la présente invention peut également comprendre la détection et/ou quantification dans le sérum dudit sujet d'anticorps dirigés contre d'autres auto-antigènes et préférentiellement l'antigène myeloperoxydase (MPO) et/ou l'antigène protéinase 3 (PR3) et/ou élastase et/ou BPI et/ou cathépsine G et/ou des antigènes nucléaires.

Selon la présente invention, le diagnostic d'une réponse immunitaire autoimmune peut également être associé une pathologie caractérisée par une souffrance tissulaire chez le sujet. Dans ce cas, et de manière préférée, la souffrance tissulaire chez le sujet est due à une nécrose, particulièrement un infarctus, une inflammation chronique ou une infection chronique.

La souffrance tissulaire est appréciée en mesurant la concentration dans différents fluides biologiques de molécules libérées lorsque les cellules souffrent (ces molécules peuvent être des enzymes telle que les transaminases ou des protéines de l'inflammation).

Un deuxième aspect de la présente invention concerne l'utilisation d'un kit de diagnostic pour la détection et/ou la quantification dans un liquide biologique d'anticorps dirigés contre l'antigène PTX3, comprenant
a) un support solide caractérisé en ce que l'antigène PTX3 est fixé sur le support solide.

Les caractéristiques du kit (support solide, antigène PTX3...) sont telles que définies précédemment pour la méthode de diagnostique.

Par liquide biologique on entend désigner selon la présente invention le sang, le sérum, le plasma, la lymphe, l'urine, la salive, le liquide céphalorachidien préférentiellement le sérum.

De manière optionnelle, le kit tel que défini précédemment comprend une solution contenant une ou des protéines de saturation qui saturent les sites réactifs du support solide.

De manière optionnelle, le kit tel que défini précédemment comprend en outre
b) une solution contenant des anticorps anti- immunoglobuline conjugués avec un marqueur

Selon la présente invention,les anticorps anti- immunoglobuline utilisés dans le kit sont préférentiellement sélectionnés dans le groupe comprenant des anti immunoglobuline G, des anti immunoglobuline A, et des anti immunoglobuline M, des anti immunoglobuline D, et des anti immunoglobuline E.

De manière optionnelle, le kit tel que défini précédemment comprend en outre
c) une solution de lavage

Par solution de lavage, on entend désigner selon la présente invention une solution saline tamponnée contenant une faible concentration d'agent détergent et ou une protéine de saturation, préférentiellement de type sérum albumine bovine ou gélatine.

Par « protéine de saturation », on entend désigner une protéine qui sature les sites réactifs du support solide.

De manière préférée, le support solide utilisé dans le kit tel que défini précédemment est une plaque ELISA et le marqueur est une enzyme.

De manière optionnelle, le kit tel que défini précédemment comprend en outre
d) une solution contenant le substrat soluble correspondant à l'enzyme.

Un quatrième aspect de la présente invention concerne l'utilisation de la méthode de diagnostic selon la présente invention ou du kit tel que défini précédemment pour identifier, avant l'apparition des signes cliniques, les sujets à risques de réponse immunitaire autoimmune.

De manière préférée, la méthode de diagnostic ou le kit tel que défini précédemment selon la présente invention sont utilisés pour suivre l'évolution de la réponse immunitaire autoimmune, prévoir les poussées évolutives de la maladie et/ou suivre l'efficacité du traitement.

Par « poussées évolutives », on entend désigner une agravation des signes cliniques.

### Légende des figures :

**Figure 1** **:** Mise en évidence d'anticorps anti-PTX3 chez des sujets présentant une immuno-fluorescence indirecte (IFI) positive, associée à des auto-anticorps anti-MPO ou à des auto-anticorps anti-PR3
**Figure 2** **:** Mise en évidence d'anticorps anti-PTX3 chez des sujets présentant une IFI positive, en l'absence d'autoanticorps anti-MPO ou à des autoanticorps anti-PR3

### Exemples :

### 1. Utilisation d'un test diagnostique permettant de rechercher des auto anticorps anti-PTX3 dans les sérums de patients.

### a. Description du test ELISA permettant de rechercher des anticorps anti-PTX3.

Des plaques ELISA *96* puits (Maxisorb® ; Nunc, Roskilde, Danemark) sont incubées ou non une nuit à 4°C avec 100 µL de PTX3 à 10 µg/mL en tampon carbonate/bicarbonate pH=9,6. Les puits sont ensuite vidés et incubés pendant 1 heure et 30 minutes avec 300 µL dune solution de sérum albumine bovine (BSA) à 1% dans du tampon phosphate salin (Phosphate saline buffer) 10 mM, pH=7,4. Les sérums de patients et de sujets sains sont dilués au 1/400 dans un tampon PBS contenant 0,5% de BSA (w:v) et 0,05% de Tween 20 (w:v) et 100 µL de cette dilution sont déposés, pour chacun des sérums, dans un puit coaté avec la PTX3 et dans un puits non coaté (qui permettra de déterminer le bruit de fond pour chaque sérum). Après une incubation de 2 h à 37°C, les plaques sont lavées 4 fois avec 200 µL de PBS contenant 1% de Tween 20 (w:v) puis incubées pendant 1 h 30 à 37°C avec 100 µL d'un anticorps anti-immunoglobuline (G, A et M) humaine couplé à la biotine (Jackson ImmunoResearch, West Grove, PA). Après 4 lavages avec 200 µL de PBS contenant 1% de Tween 20 (w:v), 100 µL d'une solution de streptavidine couplée à la peroxydase est incubée pendant 1 heure à 37°C (solution commerciale diluée au 1/1000 ; BD Pharmingen, San José, CA). Après 4 lavages en PBS contenant 1% de Tween 20 (w:v), les anticorps fixés sont détectés en utilisant le substrat ABTS (Sigma, St Louis, CA) préparé extemporanément. La lecture de la plaque ELISA se fait à l'aide d'un fluoromètre (λ=405 nm avec une référence à λ =620 nm) ; les résultats sont exprimés en valeur de densité optique (DO).

Un exemple de résultats bruts obtenus après lecture est présenté ci-dessous. La reproductibilité des résultats a été vérifiée par la réalisation de manipulations successives.

De façon générale, les résultats ont été analysés de la façon suivante :
- Pour chaque plaque ELISA, 20 à 30 sérums de sujets sains ont été testés en parallèle avec 20 à 30 sérums de patients.
- Pour chaque sérum, la valeur de DO obtenue sur le puits non coaté (bruit de fond BSA) a été soustraite à la valeur de DO obtenue avec puits coaté avec la molécule PTX3 afin d'obtenir une DO spécifique,
- Les valeurs de DO spécifiques obtenues avec les sérums de sujets sains ont été additionnées afin de déterminer la moyenne et la déviation standard. Une valeur seuil définie comme la moyenne plus 2 déviations standard est calculée pour chaque plaque ELISA (mean ± 2 s.d.),
- Les valeurs de DO spécifiques des sérums de patients sont considérées comme positives lorsqu'une valeur de DO supérieure à la valeur seuil moyenne + 2 sd est obtenue.

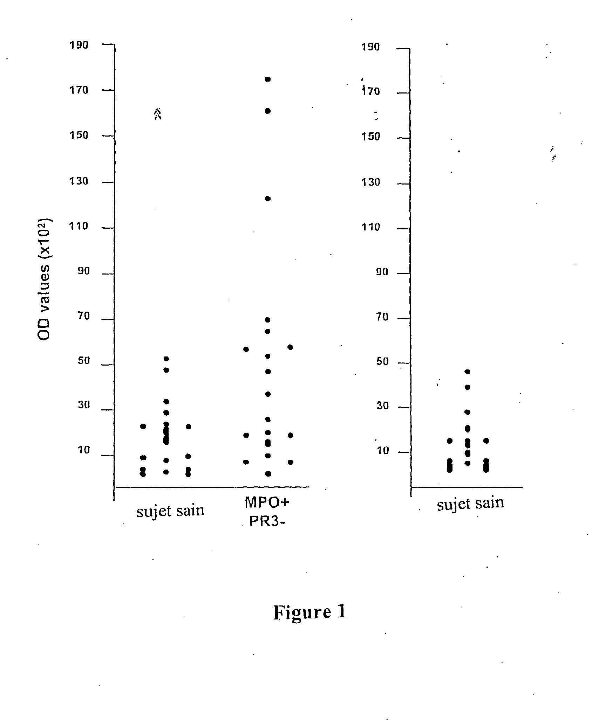

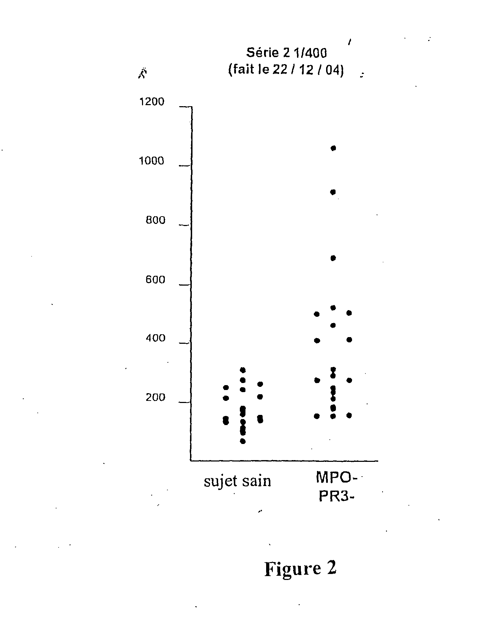

Dans les exemples suivants, des anticorps anti-PTX3 ont été recherchés chez des patients présentant des auto anticorps anti-neutrophiles (IFI+, correspondant à des ANCA+) ; 3 populations ont été analysées :
- des patients ANCA+, MPO+ (PR3-) exemple a et Figure 1 schéma de droite
- des patients ANCA+ PR3+ (MPO-) exemple b et Figure 1 schéma de gauche
- des patients ANCA+ PR3- MPO- exemple c et Figure 2.
- exemple d : parmi les patients PR3- et MPO-, nous avons ré-analysé les résultats précédents (exemple c) en fonction de la présence d'auto anticorps dirigés contre d'autres spécificités
- présentant des auto anticorps divers

### b. Présence d'anticorps anti-PTX3 chez les patients IFI + MPO+ (PR3-)

Les sérums de 22 sujets présentant des auto anticorps anti-polynucléaires (IFI+) et des anticorps anti-MPO et de 23 sujets sains ont été testés comme décrit ci-dessus. La valeur seuil (mean + 2 s.d.) est de 0.037 - 9 sérums de patients sont supérieurs à la valeur seuil. Les résultats obtenus sont présentés dans la Figure 1A.

### c. Présence d'anticorps anti-PTX3 chez les patients IFI + PR3+ (MPO-)

Les sérums de 22 sujets présentant des auto anticorps-polynucléaires (IFI+) et des anticorps anti-PR3 et de 23 sujets sains ont été testés comme décrit ci-dessus. La valeur seuil (mean + 2 s.d.) est de 0.04 - 11 sérums de patients sont supérieurs à la valeur seuil. Les résultats obtenus sont présentés dans la Figure 1B.

### d. Présence d'anticorps anti-PTX3 chez les patients IFI + MPO- PR3-

Les sérums de 21 sujets présentant des auto anticorps-polynucléaires (IFI+) et de 23 sujets sains ont été testés comme décrit ci-dessus.

La valeur seuil (mean + 2 s.d.) est de 0.3 - 9 sérums de patients sont supérieurs à la valeur seuil. Les résultats obtenus sont présentés dans la Figure 2.

### e. Analyse de la fréquence d'anticorps anti-PTX3 chez les patients ANCA+ MPO- PR3-

Chez les patients IFI+ MPO- et PR3- (n = 131), la recherche d'auto anticorps dirigés contre d'autres spécificités (Bactericidal permeability increasing protein [BPI]), cathepsine G et élastase) a été réalisée en utilisant un kit ELISA commercial (ANCAprofil, Euroimmun, XX). En parallèle, la présence dans ces sérums d'anticorps anti-PTX3 a été recherchée par ELISA, comme décrit précédemment.

Les résultats obtenus sont schématisés ci-dessous. Ils montrent que 72 % des patients ayant une IFI+ qui ne peut être confirmée à l'heure actuelle par des kits commerciaux utilisés en routine (permettant de détecter la présence d'auto anticorps contre les antigènes suivants : MPO, PR3, BPI, cathepsine G et élastase) présentent des anticorps anti-PTX3.

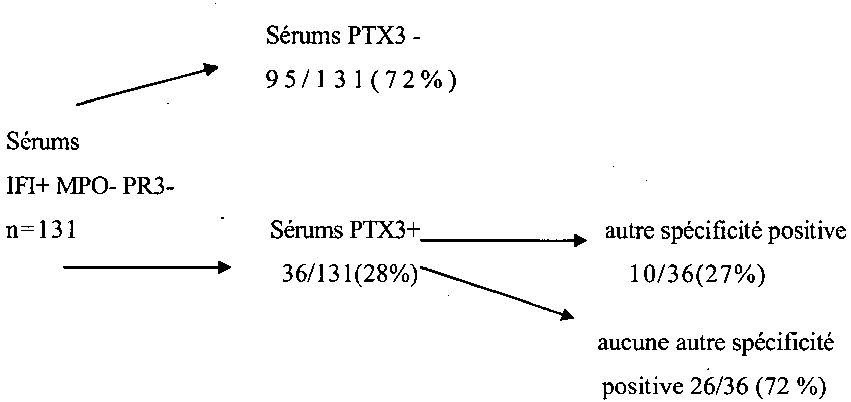

### f. Anti-PTX3 chez les sujets présentant des auto anticorps dirigés contre différents auto antigènes

La recherche d'auto anticorps anti-PTX3 a été recherchée par ELISA comme décrit précédemment dans les sérums de patients présentant différentes pathologies auto immunes (et en particulier des pathologies systémiques). Les sérums testés ont été définis sur la base de la présence :
d'auto anticorps anti-SSA : ces auto anticorps sont dirigés contre l'antigène nucléaire SSA ; ils sont retrouvés dans le syndrome de Gougerot-Sjögren (syndrome sec), isolé ou associé à une autre connectivité.
d'un test de FARR positif : qui signe la présence d'anticorps anti-ADN natif. Ces anticorps sont présents dans 90% des lupus érythémateux disséminé (LED)
d'auto anticorps antipeptide cyclique citrulliné (anti-CCP) : les anticorps anti-CCP sont un outil diagnostic très spécifique de la polyarthrite rhumatoïde,
d'anticorps *anti-Saccharomyces cerevisiae* (ASCA) : les ASCA sont dirigés contre un épitope de structure (Mana-1,2Man) ₙα-1-3Man, n = ou > 1 et sont reconnus comme associé à la maladie de Crohn.

Les résultats obtenus ont été résumés dans le tableau I.

**Tableau I : Les sérums présentant des autoanticorps (anti-SSA, anti-ADN natif, anti-Saccharomyces cerevisiae [ASCA], anti-CCP) ont été dilués au 1/400 comme décrit précédemment et testé en ELISA parallèlement à des sérums de sujets sains.**

| **Caractéristiques des sérums : présence de** | **Nombre de sérums testés :** | **Nombre de sérums présentant des anticorps anti-PTX3** | **Fréquence de sérums présentant des anticorps anti-PTX3** |
|---|---|---|---|
| Anti-SSA | 11 | 2 | 2/11 |
| Anti-ADN natif | 10 | 4 | 4/10 |
| ASCA | 10 | 3 | 3/10 |
| Anti-CCP | 10 | 1 | 1/10 |

### Bibliographie

► Abderrahim-Ferkoune A, Bezy O, Chiellini C, Maffei M, Grimaldi P, Bonino F, Moustaid-Moussa N, Pasqualini F, Mantovani A, Ailhaud G, Amri EZ. Characterization of the long pentraxin PTX3 as a TNFalpha-induced secreted protein of adipose cells. J Lipid Res. 2003, 44:994-1000.
► Agrawal A, Volanakis JE. Probing the Clq-binding site on human C-reactive protein by site-directed mutagenesis. J Immunol. 1994, 152:5404-10.
► Alles VV, Bottazzi B, Peri G, Golay J, Introna M, Mantovani A. Inducible expression of PTX3, a new member of the pentraxin family, in human mononuclear phagocytes. Blood. 1994, 84:3483-93.
► Bijl M, Horst G, Bijzet J, Bootsma H, Limburg PC, Kallenberg CG. Serum amyloid P component binds to late apoptotic cells and médiates their uptake by monocyte-derived macrophages. Arthritis. Rheum. 2003, 48:248-54.
► Bottazzi B, Vouret-Craviari V, Bastone A, De Gioia L, Matteucci C, Peri G, Spreafico F, Pausa M, D'Ettorre C, Gianazza E, Tagliabue A, Salmona M, Tedesco F, Introna M, Mantovani A. Multimer formation and ligand récognition by the long pentraxin PTX3. Similarities and differences with the short pentraxins C-reactive protein and serum amyloid P component. J. Biol. Chem. 1997, 272:32817-23.
► Breviario F, dAniello EM, Golay J, Peri G, Bottazzi B, Bairoch A, Saccone S, Marzella R, Predazzi V, Rocchi M, et al. Interleukin-1-inducible genes in endothelial cells. Cloning of a new gene related to C-reactive protein and serum amyloid P component. J Biol Chem. 1992, 267:22190-7.
► Dias AA, Goodman AR, Dos Santos JL, Gomes RN, Altmeyer A, Bozza PT, Horta MF, Vilcek J, Reis LF. TSG-14 transgenic mice have improved survival to endotoxemia and to CLP-induced sepsis. J. Leuk. Biol. 2001, 69:928-36.
► Diniz SN, Nomizo R, Cisalpino PS, Teixeira MM, Brown GD, Mantovani A, Gordon S, Reis LF, Dias AA. PTX3 fonction as an opsonin for the dectm-1-dependent intemalization of zymosan by macrophages. J Leukoc Biol. 2004 75, 649-56.
► Doni A, Peri G, Chieppa M, Allavena P, Pasqualini F, Vago L, Romani L, Garlanda C, Mantovani A. Production of the soluble pattern recognition receptor PTX3 by myeloid, but not plasmacytoid, dendritic cells. Eux J Immunol. 2003, 33:2886-93.
► Garlanda C, Bottazzi B, Bastone A, Mantovani A. Pentraxins at the crossroads Between Imiate Immunity, Inflammation, Matrix Deposition and Female Fertility. Annu Rev Immunol. 2005, 23: 337-366.
► Garlanda C, Hirscli E, Bozza S, Salustri A, De Acetis M, Nota R, Maccagno A, Riva F, Bottazzi B, Peri G, Doni A, Vago L, Botto M, De Santis R, Carminati P, Siracusa G, Altruda F, Vecchi A, Romani L, Mantovani A. Non-redundant role of the long pentraxin PTX3 in anti-fungal innate immune response. Nature. 2002, 420:182-6.
► Gershov D, Kim S, Brot N, Elkon KB. C-Reactive protein binds to apoptotic cells, protects the cells from assembly of the terminal complement components, and sustains an antiinflammatory innate immune response: implications for systemic autoimmunity. J. Exp. Med. 2000, 192:1353-64.
► Goodman AR, Levy DE, Reis LF, Vilcek J. Differential regulation of TSG-14 expression in murine fibroblasts and peritoneal macrophages. J Leukoc Biol. 2000, 67:387-95,
► Hind CR, Collins PM, Renn D, Cook RB, Caspi D, Marilyn L Baltz, and Pepys MB, Binding specificity of serum amyloid P component for the pyruvate acetal of galactose. J Exp Med. 1984 Apr 1;159(4):1058-69.
► Janeway CA Jr, Medzhitov R., Innate immune recognition. Annu Rev Immunol. 2002;20:197-216. Epub 2001 Oct 4. Review.
► Lee GW, Lee TH, Vilcek J. TSG-14, a tumor necrosis factor- and IL-1-inducible protein. is a novel member of the pentaxin family of acute phase proteins. J Immunol. 1993; 150:1804-12.
► Matsushita M, Fujita T. The role of ficolins in innate immunity. Immunobiology. 2002, 205:490-7. Nauta AJ, Bottazzi B, Mantovani A, Salvatori G, Kishore U, Schwaeble WJ, Gingras AR, Tzirna S, Vivanco F, Egido J, Tijsma O, Hack EC, Daha MR, Roos A. Biochemical and functional characterization of the interaction between pentraxin 3 and Clq. Eur J Immunol. 2003, 33:465-73.
► Medzhitov R, Janeway C Jr., Innate immunity, N Engl J Med. 2000 Aug 3;343(5):338-44. Review.
► Nauta AJ, de Haij S, Bottazzi B, Mantovani A, Bornas MC, Aten J, Rastaldi MP, Daha MR, van Kooten C, Roos A. Human renal epithelial cells produce the long pentraxin PTX3. Kidney Int. 2005, 67:543-53.
► Rovere P, Peri G, Fazzini F, Bottazzi B, Doni A, Bondanza A, Zinunermann VS, Garlanda C, Fascio U, Sabbadini MG, Rugarli C, Mantovani A, Manfredi AA. The long pentraxin PTX3 binds to apoptotic cells and regulates their clearance by antigen-presenting dendritic cells. Blood. 2000, 96:4300-6.
► Van de Wetering JK, van Golde LM, Batenburg JJ. Collectins: players of the innate immune system. Eux. J. Biochem. 2004, 271:1229-49.
► Vouret-Craviari V, Matteucci C, Peri G, Poli G, Introna M, Mantovani A. Expression of a long pentraxin, PTX3, by monocytes exposed to the mycobacterial cell wall component lipoarabinomannan. Infect Immun. 1997, 65:1345-50.

## Revendications

1. Méthode de diagnostic in vitro d'une réponse immunitaire autoimmune chez un sujet par détection dans un liquide biologique dudit sujet d'anticorps dirigés contre l'antigène pentraxine 3 (PTX3) **caractérisée en ce que** on détermine dans le sérum du sujet la présence d'anticorps dirigés contre l'antigène PTX3 (anticorps anti-PTX3) et qu'on en conclue l'existence d'une réponse immunitaire autoimmune chez le sujet.

2. Méthode selon la revendication 1 **caractérisée en ce que** on détermine dans un liquide biologique du sujet le taux d'anticorps dirigés contre l'antigène PTX3 et qu'en le comparant avec le taux d'anticorps dirigés contre l'antigène PTX3 de référence, on détermine l'existence d'une réponse immunitaire autoimmune chez le sujet.

3. Méthode selon l'une quelconque des revendications 1 ou 2 **caractérisée en ce que** la présence et/ou le taux d'anticorps dirigés contre PTX3 est déterminé par la mise en évidence d'une liaison entre l'antigène PTX3 et l'anticorps anti-PTX3.

4. Méthode selon l'une quelconque des revendications 2 à 3 **caractérisée en ce que** le taux d'anticorps anti-PTX3 de référence est le taux d'anticorps anti-PTX3 obtenu à partir du sérum d'un sujet sain, d'un ensemble de sérum de sujets sains ou défini de façon arbitraire par tous moyens qui permettront de reproduire la valeur moyenne obtenue avec un pool de sujet sains.

5. Méthode selon l'une quelconque des revendications 1 à 4 **caractérisée en ce que** les antigènes PTX3 sont choisis parmi la PTX3 entière d'origine humaine, animale, ou synthétique, un ou plusieurs fragments de PTX3 d'origine humaine, animale ou synthétique, et une molécule homologue à PTX3, préférentiellement de la famille des pentraxines, et/ou présentant des homologies de séquences primaires, secondaires ou tertiaires importantes.

6. Méthode selon l'une quelconque des revendications 1 à 5 **caractérisée en ce que** le diagnostic d'une réponse immunitaire autoimmune est associé à l'existence ou à la prédiction d'une maladie auto-immune chez le sujet.

7. Méthode selon la revendication 6 **caractérisée en ce que** les maladies auto-immunes sont sélectionnées dans le groupe comprenant le syndrome de Gougerot-Sjögren, le diabète de type 1, la gammapathie monoclonale, la granulomatose de Wegener, le lupus érythémateux disséminé, la maladie athéromateuse, la maladie de Crohn, la maladie de Horton, la maladie de Reiter (syndrome conjonctivo-urétro-synovial), la polyarthrite rhumatoïde, la rectocolite hémorragique, le rhumatisme psoriasique, la sarcoïdose, la sclérodermie, la sclérose en plaques, et les dermatites bulleuses auto-immunes, la maladie de Basedow (hyperthyroïdie), thyroïdite chronique de Hashimoto (hypothyroïdie), le symdrôme de Goodpasture, le pemphigus, la myasthénie, le diabète par insulinorésistance, l'anémie hémolytique auto-immune, la purpura thrombocytopénique auto-immun, polymyosite et dermatomyosite, l'aménie de Biermer, la glomérulonéphrite, certaines stérilités, périartérite noueuse et le syndrome de Churg et Strauss, la maladie de Still, la polychondrite atrophiante, la maladie de Behçet, la spondylarthrite.

8. Méthode selon l'une des revendications 1 à 7 **caractérisée en ce qu'**elle comprend également la détection et/ou quantification dans le sérum dudit sujet d'anticorps dirigés contre l'antigène myeloperoxydase (MPO) et/ou l'antigène protéinase 3 (PR3) et/ou élastase et/ou BPI et/ou cathepsine G et/ou des antigènes nucléaires.

9. Méthode selon l'une quelconque des revendications 1 à 5 **caractérisée en ce que** le diagnostic d'une réponse immunitaire autoimmune est associé à une pathologie **caractérisée par** une souffrance tissulaire chez le sujet due à une nécrose, particulièrement un infarctus, une inflammation chronique ou une infection chronique.

10. Utilisation d'un kit de diagnostic in vitro d'une réponse autoimmune chez un sujet pour la détection et/ou la quantification dans un liquide biologique d'anticorps dirigés contre l'antigène PTX3, ledit kit comprenant :
a) un support solide **caractérisé en ce que** l'antigène PTX3 est fixé sur le support solide.

11. Utilisation selon la revendication 10 **caractérisée en ce que** ledit kit comprend en outre :
b) une solution contenant des anticorps anti- immunoglobuline conjugués avec un marqueur.

12. Utilisation selon la revendication 11 **caractérisée en ce que** le support solide est une plaque ELISA et le marqueur est une enzyme, ledit kit comprenant en outre une solution contenant le substrat soluble correspondant à l'enzyme.

13. Utilisation de la méthode de diagnostic selon les revendications 1 à 9 ou du kit tel que défini selon les revendications 10 à 12 pour identifier, avant l'apparition des signes cliniques, les sujets présentant un risque de développer une maladie autoimmune.

14. Utilisation de la méthode de diagnostic selon les revendication 1 à 9 ou du kit tel que défini selon les revendication 10 à 12 pour suivre l'évolution de la réponse immunitaire autoimmune, prévoir les poussées évolutives de la maladie et/ou suivre l'efficacité du traitement.

## Patentansprüche

1. Verfahren zur in-vitro-Diagnose einer Autoimmunantwort bei einem Subjekt durch Nachweis von Antikörpern, die gegen das Pentraxin-3 (PTX3)-Antigen gerichtet sind, in einer biologischen Flüssigkeit des Subjekts, **dadurch gekennzeichnet, dass** man die Anwesenheit von Antikörpern, die gegen das PTX3-Antigen gerichtet sind (Anti-PTX3-Antikörper), im Serum des Patienten bestimmt und dass man daraus auf das Vorhandensein einer Autoimmunantwort bei dem Subjekt schließt.

2. Verfahren nach Anspruch 1, **dadurch gekennzeichnet, dass** man den Gehalt an Antikörpern, die gegen das PTX3- Antigen gerichtet sind, in einer biologischen Flüssigkeit des Subjekts bestimmt und dass man, indem man ihn mit dem Gehalt an Antikörpern vergleicht, die gegen ein Referenz-PTX3-Antigen gerichtet sind, das Vorhandensein einerAutoimmunantwort bei dem Subjekt bestimmt.

3. Verfahren nach irgendeinem der Ansprüche 1 oder 2, **dadurch gekennzeichnet, dass** man die Anwesenheit und/oder der Gehalt von Antikörpern, die gegen PTX3 gerichtet sind, durch den Nachweis einer Bindung zwischen dem PTX3-Antigen und dem Anti-PTX3-Antikörper bestimmt.

4. Verfahren nach irgendeinem der Ansprüche 2 bis 3, **dadurch gekennzeichnet, dass** der Gehalt an Referenz-Anti-PTX3-Antikörper der Gehalt von Anti-PTX3-Anti-körpern ist, der aus dem Serum eines gesunden Subjekts, einem Serumbestand von gesunden Subjekten erhalten wurde oder auf willkürliche Weise durch alle Mittel definiert wurde, die es erlauben, den mittleren Wert zu reproduzieren, der bei einem Pool von gesunden Subjekten erhalten wird.

5. Verfahren nach irgendeinem der Ansprüche 1 bis 4, **dadurch gekennzeichnet, dass** die PTX3-Antigene ausgewählt sind aus vollständigem PTX3 menschlichen, tierischen oder synthetischen Ursprungs, einem oder mehreren Fragmenten von PTX3 menschlichen, tierischen oder synthetischen Ursprungs und einem zu PTX3 homologen Molekül vorzugsweise aus der Familie der Pentraxine und/oder bedeutende primäre, sekundäre oder tertiäre Sequenzhomologien aufweisend.

6. Verfahren nach irgendeinem der Ansprüche 1 bis 5, **dadurch gekennzeichnet, dass** die Diagnose einer Autoimmunantwort mit dem Vorhandensein oder der Vorhersage einer Autoimmunkrankheit bei dem Subjekt verbunden ist.

7. Verfahren nach Anspruch 6, **dadurch gekennzeichnet, dass** die Autoimmunkrankheiten ausgewählt sind aus der Gruppe, welche umfasst: das Gougerot-Sjörgen-Syndrom, Typ-1-Diabetes, monoklonale Gammopathie, Wegener-Granulomatose, disseminierten Lupus erythematodes,-atheromatöse Erkrankung, Morbus Crohn, das Horton-Syndrom, das Reiter-Syndrom (uretro-okulo-synoviales Syndrom), rheumatoide Polyarthritis, hämorrhagische Rektokolitis, Arthritis psoriatica, Sarkoidose, Sklerodermie, Multiple Sklerose und bullöse Autoimmun-Dermatitiden, Morbus Basedow (Hyperthyreose), chronische Hashimoto-Thyreose (Hypothyreose), das Goodpasture-Syndrom, Pemphigus, Myastenie, insulinresistenten Diabetes, hämolytische Autoimmun-Anämie, autoimmune thrombozytopenische Purpura, Polymyositis und Dermatomyositis, Biermer-Anämie, Glomerulonephritis, gewisse Unfruchtbarkeiten/Zeugungsunfähigkeiten, Periarteritis nodosa und das Churg-Strauss-Syndrom, Morbus Still, atrophierende Polychondritis, die Behçet-Krankheit, Spondylarthritis.

8. Verfahren nach einem der Ansprüche 1 bis 7, **dadurch gekennzeichnet, dass** es auch den Nachweis und/oder die quantitative Bestimmung von Antikörpern, die gegen das Myeloperoxydase (MPO)-Antigen und/oder gegen das Proteinase-3 (PR3)-Antigen und/oder gegen Elastase und/oder gegen PPI und/oder gegen Cathepsin G und/oder gegen nukleäre Antigene gerichtet sind, im Serum des Subjekts umfasst.

9. Verfahren nach irgendeinem der Ansprüche 1 bis 5, **dadurch gekennzeichnet, dass** die Diagnose einer Autoimmunantwort mit einer Krankheit verbunden ist, die durch ein Gewebeleiden bei dem Subjekt aufgrund einer Nekrose, insbesondere eines Infarkts, einer chronischen Entzündung oder einer chronischen Infektion gekennzeichnet ist.

10. Verwendung eines Kits zur in-vitro-Diagnose einer Autoimmunantwort bei einem Subjekt für den Nachweis und/oder die quantitative Bestimmung von Antikörpern, die gegen das PTX3-Antigen gerichtet sind, in einer biologischen Flüssigkeit, wobei das Kit umfasst:
a) einen festen Träger, **dadurch gekennzeichnet, dass** das PTX3-Antigen auf dem festen Träger befestigt ist.

11. Verwendung nach Anspruch 10, **dadurch gekennzeichnet, dass** das Kit ferner umfasst:
b) eine Lösung, die mit einem Marker konjugierte Anti-Immunglobulin-Antikörper enthält.

12. Verwendung nach Anspruch 11, **dadurch gekennzeichnet, dass** der feste Träger eine ELISA-Platte ist und der Marker ein Enzym ist, wobei das Kit ferner eine Lösung umfasst, die das lösliche dem Enzym entsprechende Substrat enthält.

13. Verwendung des Diagnoseverfahrens nach den Ansprüchen 1 bis 9 oder des Kits, wie gemäß den Ansprüchen 10 bis 12 definiert, um vor dem Auftreten von klinischen Anzeichen Subjekte zu identifizieren, die ein Risiko aufweisen, eine Autoimmunkrankheit zu entwickeln.

14. Verwendung des Diagnoseverfahrens nach den Ansprüchen 1 bis 9 oder des Kits, wie gemäß den Ansprüche 10 bis 12 definiert, um die Entwicklung der Autoimmunantwort zu verfolgen, fortschreitende Krankheitsschübe der Krankheit vorherzusehen und/oder die Wirksamkeit der Behandlung zu verfolgen.

## Claims

1. A Method for *in vitro* diagnosis of an autoimmune immune response in a subject by detection in a biological fluid of said subject of antibodies directed against the pentraxin 3 antigen (PTX3) **characterized in that** the presence of antibodies directed against the PTX3 antigen (anti-PTX3 antibodies) is determined in the serum of said subject and the existence of an autoimmune immune response in the subject is concluded on this basis.

2. The method according to claim 1, **characterized in that** the quantity of antibodies directed against PTX3 antigen is determined in a biological fluid of the subject and the existence of an autoimmune immune response in the subject is established through comparison with the quantity of antibodies directed against reference PTX3 antigen.

3. The method according to any one of claims 1 or 2, **characterized in that** the presence and/or quantity of antibodies directed against PTX3 is determined by detection of binding between the PTX3 antigen and the anti-PTX3 antibody.

4. The method according to any one of claims 2 to 3, **characterized in that** the quantity of reference anti-PTX3 antibodies is the quantity of anti-PTX3 antibodies obtained from the serum of a healthy subject, from a set of serum from healthy subjects or defined in an arbitrary manner by any means which allows reproduction of the mean value obtained with a pool from healthy subjects.

5. The method according to any one of claims 1 to 4, **characterized in that** the PTX3 antigens are chosen from whole PTX3 of human, animal or synthetic origin, one or more PTX3 fragments of human, animal or synthetic origin, and PTX3 homologue molecules, preferentially from the pentraxins family and/or presenting substantial homology with the primary, secondary or tertiary sequences.

6. The method according to any one of claims 1 to 5, **characterized in that** the diagnosis of an autoimmune immune response is associated with the existence or prediction of an autoimmune disease in the subject.

7. The method according to claim 6, **characterized in that** the autoimmune diseases are selected from the group comprising Gougerot-Sjögren syndrome, type 1 diabetes, monoclonal gammapathy, Wegener's granulomatosis, disseminated lupus erythematosus, atheromatous disease, Crohn's disease, Horton's disease, Reiter's disease (conjunctivo-uretro-synovial syndrome), rheumatoid arthritis, haemorrhagic recto-colitis, psoriatic rheumatism, sarcoidosis, sclerodermy, multiple sclerosis and autoimmune bullous dermatoses, Basedow's disease (hyperthyroidism), Hashimoto's chronic thyroiditis (hypothyroidism), Goodpasture's syndrome, pemphigus, myasthenia, insulin resistant diabetes, autoimmune haemolytic anaemia, autoimmune thrombocytopenic purpura, polymyositis and dermatomyositis, Biermer's anaemia, glomerulonephritis, certain sterile diseases, periarteritis nodosum and Churg-Strauss syndrome, Still's disease, atrophying polychondritis, Behçet's disease and spondylarthritis.

8. The method according to one of claims 1 to 7, **characterized in that** it also comprises detection and/or quantification in the serum of said subject of antibodies directed against the myeloperoxidase antigen (MPO) and/or proteinase 3 antigen (PR3) and/or elastase and/or BPI and/or cathepsin G and/or nuclear antigens.

9. The method according to any one of claims 1 to 5, **characterized in that** the diagnosis of an autoimmune immune response is associated with a pathology **characterized by** tissue damage in the subject due to necrosis, particularly infarction, chronic inflammation or chronic infection.

10. Use of an in vitro diagnostic kit of an autoimmune response in a subject for detection and/or quantification in a biological fluid of antibodies directed against the PTX3 antigen comprising:
a) a solid support **characterized in that** the PTX3 antigen is fixed to the solid support.

11. Use according to claim 10, **characterized in that** said kit further comprises:
b) a solution containing anti-immunoglobulin antibodies conjugated to a marker.

12. Use according to claim 11, **characterized in that** the solid support is an ELISA plate and the marker is an enzyme, said kit further comprises a solution containing the soluble substrate corresponding to the enzyme.

13. Use of the diagnostic method according to claims 1 to 9 or kit according to claims 10 to 12, to identify, prior to the appearance of clinical symptoms, subjects who run the risk of developing an autoimmune disease.

14. Use of the diagnostic method according to claims 1 to 9 or kit according to claims 10 to 12, to monitor the evolution of an autoimmune immune response, predict progression of the disease and/or monitor the efficacy of treatment.
